# EUROPÄISCHE PATENTSCHRIFT

(11) **EP 0 873 300 B1**
(45) Veröffentlichungstag und Bekanntmachung des Hinweises auf die Patenterteilung: **23.08.2000**
(21) Anmeldenummer: 96932496.1
(22) Anmeldetag: 11.09.1996
(51) Int. Cl.: C07C 209/72

(54) **VERFAHREN ZUR HYDRIERUNG VON AROMATISCHEN VERBINDUNGEN, IN DENEN MINDESTENS EINE AMINOGRUPPE AN EINEN AROMATISCHEN KERN GEBUNDEN IST**
METHOD OF HYDROGENATING AROMATIC COMPOUNDS IN WHICH AT LEAST ONE AMINO GROUP IS BONDED TO AN AROMATIC NUCLEUS
PROCEDE D'HYDROGENATION DE COMPOSES AROMATIQUES, DANS LESQUELS AU MOINS UN GROUPE AMINO EST LIE A UN NOYAU AROMATIQUE

(30) Priorität: 12.09.1995 DE 19533718
(43) Veröffentlichungstag der Anmeldung: 28.10.1998
(73) Patentinhaber: BASF AKTIENGESELLSCHAFT, 67056 Ludwigshafen (DE)
(72) Erfinder: RÜTTNER, Heinz, D-67126 Hochdorf-Assenheim (DE); RÜHL, Thomas, D-67227 Frankenthal (DE); BREITSCHEIDEL, Boris, D-36043 Fulda (DE); HENKELMANN, Jochem, D-68165 Mannheim (DE); HENNE, Andreas, D-67433 Neustadt (DE); WETTLING, Thomas, D-67117 Limburgerhof (DE)
(74) Vertreter: Isenbruck, Günter, Dr.
(86) Internationale Anmeldenummer: EP9603983
(87) Internationale Veröffentlichungsnummer: WO9710202

(56) Entgegenhaltungen:
- EP-A- 0 501 265
- US-A- 5 360 934

## Beschreibung

Die vorliegende Erfindung betrifft ein Verfahren zur Hydrierung von aromatischen Verbindungen, in denen mindestens eine Aminogruppe an einen aromatischen Kern gebunden ist.

Die vorliegende Erfindung betrifft insbesondere ein Verfahren zur Hydrierung von aromatischen Aminen und Diaminen. Die ein- oder mehrkernigen gegebenenfalls substituierten aromatischen Amine und Diamine werden dabei zu den entsprechenden cycloaliphatischen Aminen und Diaminen hydriert mit Hilfe von Katalysatoren, die Ruthenium und gegebenenfalls mindestens ein weiteres Metall der I-, VII- oder VIII-Nebengruppe auf einem Träger enthalten.

Cycloaliphatische Amine, insbesondere gegebenenfalls substituierte Cyclohexylamine und Dicyclohexylamine, finden Verwendung zur Herstellung von Alterungsschutzmitteln für Kautschuke und Kunststoffe, als Korrosionsschutzmittel sowie als Vorprodukte für Pflanzenschutzmittel und Textilhilfsmittel. Cycloaliphatische Diamine werden zudem bei der Herstellung von Polyamid- und Polyurethanharzen eingesetzt und finden weiterhin Verwendung als Härter für Epoxyharze.

Es ist bekannt, cycloaliphatische Amine oder Diamine durch katalytische Hydrierung der entsprechenden ein- oder mehrkernigen aromatischen Amine oder Diamine herzustellen. Die Hydrierung von aromatischen Aminen und Diaminen zu den entsprechenden cycloaliphatischen Aminen und Diaminen in Gegenwart von Hydrierungskatalysatoren, insbesondere auf Trägern aufgebrachten Katalysatoren, ist vielfach beschrieben.

Als Katalysatoren wurden beispielsweise Raney-Kobalt mit basischen Zusätzen verwendet (JP 43/3180), Nickel-Katalysatoren (US 4,914,239, DE-PS 80 55 18), Rhodium-Katalysatoren (BE 739376, JP 7019901, JP 7235424), wie auch Palladium-Katalysatoren (US 3,520,928, EP 501 265, EP 53 181, JP 59/196843). Ebenfalls verwendet wurden Ruthenium-Katalysatoren (US 3,697,449, US 3,636,108, US 2,822,392, US 2,606,925, EP 501 265, EP 324 984, EP 67 058, DE 21 32 547, DE-OS 11 06 319).

Aus der DE 21 32 547 ist ein Verfahren zur Hydrierung ein- oder mehrkerniger aromatischer Diamine zu den entsprechenden cycloaliphatischen Aminen bekannt, das in Gegenwart von suspendierten Ruthenium-Katalysatoren ausgeführt wird.

In der EP 67 058 ist ein Verfahren zur Herstellung von Cyclohexylamin durch katalytische Hydrierung des entsprechenden aromatischen Amins beschrieben. Als Katalysator wird Rutheniummetall in einer fein verteilten Form auf aktivierten Aluminiumpellets verwendet. Nach vier Rückführungen begann der Katalysator seine Wirksamkeit zu verlieren.

Eine Aufgabe der vorliegenden Erfindung ist die Bereitstellung eines Verfahrens zur Hydrierung von Verbindungen, in denen mindestens eine Aminogruppe an einen aromatischen Kern gebunden ist, wobei das Verfahren insbesondere ohne Katalysatorabtrennung, -aufarbeitung und -rückführung durchgeführt werden kann.

Eine weitere Aufgabe der Erfindung ist die Bereitstellung eines Verfahrens zur Hydrierung von aromatischen Verbindungen, in denen mindestens eine Aminogruppe an einen aromatischen Kern gebunden ist, zu den entsprechenden cycloaliphatischen Verbindungen, wobei nur ein minimaler Nebenproduktanteil anfällt.

Eine weitere Aufgabe der vorliegenden Erfindung ist die Bereitstellung eines Verfahrens zur Hydrierung von aromatischen Verbindungen, in denen mindestens eine Aminogruppe an einen aromatischen Kern gebunden ist, wobei hohe Katalysatorbelastungen und lange Katalysatorstandzeiten ermöglicht werden.

Gelöst werden die vorstehenden Aufgaben durch ein Verfahren zur Hydrierung von aromatischen Verbindungen, in denen mindestens eine Aminogruppe an einen aromatischen Kern gebunden ist, wobei man mindestens eine dieser Verbindungen in Gegenwart eines Katalysators mit freiem Wasserstoff in Berührung bringt, wobei der Katalysator Ruthenium und gegebenenfalls mindestens ein Metall der I-, VII- oder VIII-Nebengruppe in einer Menge von 0,01 bis 30 Gew.-%, bezogen auf das Gesamtgewicht des Katalysators, aufgebracht auf einen Träger, umfaßt, wobei der Träger einen mittleren Porendurchmesser von mindestens 0,1 µm und eine Oberfläche von höchstens 15 m²/g aufweist und ausgewählt ist aus Aktivkohle, Siliciumcarbid, Aluminiumoxid, Siliciumdioxid, Titandioxid, Zirkoniumdioxid, Magnesiumoxid, Zinkoxid oder deren Gemischen, vorzugsweise Aluminiumoxid oder Zirkoniumdioxid.

Die Aufgabe wird zudem gelöst durch einen Katalysator, der Ruthenium und gegebenenfalls mindestens ein Metall der I-, VII- oder VIII-Nebengruppe in einer Menge von 0,01 bis 30 Gew.-%, bezogen auf das Gesamtgewicht des Katalysators, aufgebracht auf einen Träger, umfaßt, wobei der Träger einen mittleren Porendurchmesser von mindestens 0,1 µm und eine Oberfläche von höchstens 15 m²/g aufweist und ausgewählt ist aus Aktivkohle, Siliciumcarbid, Aluminiumoxid, Siliciumdioxid, Titandioxid, Zirkoniumdioxid, Magnesiumoxid, Zinkoxid oder deren Gemischen, vorzugsweise Aluminiumoxid oder Zirkoniumdioxid.

Mit dem erfindungsgemäßen Verfahren können die aromatischen Verbindungen, in denen mindestens eine Aminogruppe an einen aromatischen Kern gebunden ist, mit hoher Selektivität zu den entsprechenden cycloaliphatischen Verbindungen hydriert werden.

Insbesondere wird vorzugsweise die Bildung von Desaminierungsprodukten, wie beispielsweise Cyclohexanen oder teilhydrierten Dimerisierungsprodukten wie Phenylcyclohexylaminen, praktisch vollständig vermieden.

Zudem können vorzugsweise hohe Katalysatorbelastungen und lange Katalysatorstandzeiten erzielt werden. Die Katalysatorbelastung ist dabei die Raum-Zeit-Ausbeute des Verfahrens, d.h. die Menge des umgesetzten Eduktes pro Zeiteinheit und pro Menge an vorliegendem Katalysator. Standzeit bedeutet die Zeit bzw. die Menge an umgesetztem Edukt, die ein Katalysator verkraftet, ohne seine Eigenschaften einzubüßen und ohne daß sich die Produkteigenschaften signifikant verändern.

### VERBINDUNGEN

Mit dem erfindungsgemäßen Verfahren können aromatische Verbindungen, in denen mindestens eine Aminogruppe an einen aromatischen Kern gebunden ist, zu den entsprechenden cycloaliphatischen Verbindungen hydriert werden. Dabei können die aromatischen Verbindungen einkernige oder mehrkernige aromatische Verbindungen sein. Die aromatischen Verbindungen enthalten mindestens eine Aminogruppe, die an einen aromatischen Kern gebunden ist. Vorzugsweise sind die aromatischen Verbindungen aromatische Amine oder Diamine. Die aromatischen Verbindungen können an dem aromatischen Kern oder den aromatischen Kernen oder an der Aminogruppe substituiert sein durch einen oder mehrere Alkyl- und/oder Alkoxyreste, vorzugsweise C₁₋₂₀-Alkyl- und/oder Alkoxyreste, besonders bevorzugt C₁₋₁₀-Alkylreste, insbesondere Methyl-, Ethyl-, Propyl-, Isopropyl-, Butyl-, Isobutyl-, tert.-Butylreste; unter den Alkoxyresten sind die C₁₋₈-Alkoxyreste bevorzugt. Der aromatische Kern oder die aromatischen Kerne sowie die Alkyl- und Alkoxyreste können gegebenenfalls durch Halogenatome, insbesondere Fluoratome, substituiert sein oder andere geeignete inerte Substituenten aufweisen.

Die aromatische Verbindung, in der mindestens eine Aminogruppe an einen aromatischen Kern gebunden ist, kann auch mehrere aromatische Kerne aufweisen, die über einen Alkylrest, vorzugsweise eine Methylengruppe, verknüpft sind. Die verknüpfende Alkylkette, vorzugsweise Methylengruppe, kann einen oder mehrere Alkylsubstituenten aufweisen, die C₁₋₂₀-Alkylreste sein können, vorzugsweise C₁₋₁₀-Alkylreste, besonders bevorzugt Methyl-, Ethyl-, Propyl-, Isopropyl-, Butyl-, sec.-Butyl- oder tert.-Butylreste sind.

Die an den aromatischen Kern gebundene Aminogruppe kann ebenfalls durch einen oder zwei der vorstehend beschriebenen Alkylreste substituiert sein.

Besonders bevorzugte Verbindungen sind Anilin, Naphthylamin, Diaminobenzole, Diaminotoluole und Bis-p-Aminophenylmethan.

### KATALYSATOREN

Die erfindungsgemäß verwendeten Katalysatoren können technisch hergestellt werden durch Auftragen von Ruthenium und gegebenenfalls mindestens einem Metall der I-, VII- oder VIII-Nebengruppe auf einen geeigneten Träger. Die Auftragung kann durch Tränken des Trägers in wäßrigen Metallsalzlösungen, wie Rutheniumsalzlösungen, durch Aufsprühen entsprechender Metallsalzlösungen auf den Träger oder durch andere geeignete Verfahren erreicht werden. Als Rutheniumsalze zur Herstellung der Rutheniumsalzlösungen wie auch als Metallsalze der I-, VII- oder VIII-Nebengruppe eignen sich die Nitrate, Nitrosylnitrate, Halogenide, Carbonate, Carboxylate, Acetylacetonate, Chlorkomplexe, Nitritokomplexe oder Aminkomplexe der entsprechenden Metalle, bevorzugt sind dabei die Nitrate und Nitrosylnitrate.

Bei Katalysatoren, die neben Ruthenium noch weitere Metalle auf den Träger aufgetragen enthalten, können die Metallsalze bzw. Metallsalzlösungen gleichzeitig oder nacheinander aufgebracht werden.

Die mit der Rutheniumsalz- bzw. Metallsalzlösung beschichteten bzw. getränkten Träger werden sodann getrocknet, vorzugsweise bei Temperaturen zwischen 100°C und 150°C, und wahlweise bei Temperaturen zwischen 200°C und 600°C calciniert.

Darauffolgend werden die beschichteten Träger aktiviert durch Behandlung der beschichteten Träger in einem Gasstrom, der freien Wasserstoff enthält, bei Temperaturen zwischen 30 und 600°C, vorzugsweise zwischen 150 und 450°C. Der Gasstrom besteht vorzugsweise aus 50 bis 100 Vol-% H₂ und 0 bis 50 Vol-% N₂.

Werden auf die Träger neben Ruthenium noch ein oder mehrere andere Metalle der I-, VII- oder VIII-Nebengruppe aufgetragen, und erfolgt das Auftragen nacheinander, so kann der Träger nach jedem Auftragen bzw. Tränken bei Temperaturen zwischen 100 und 150°C getrocknet werden und wahlweise bei Temperaturen zwischen 200 und 600°C calciniert werden. Dabei kann die Reihenfolge, in der die Metallsalzlösungen aufgetragen oder aufgetränkt werden, beliebig gewählt werden.

Werden neben Ruthenium ein oder mehrere weitere Metalle der I-, VII- oder VIII-Nebengruppe auf den Träger aufgebracht, so werden vorzugsweise Kupfer, Rhenium, Kobalt, Nickel oder deren Gemische verwendet.

Die Rutheniumsalzlösung oder Metallsalzlösung wird in einer solchen Menge auf den oder die Träger aufgebracht, daß 0,01 bis 30 Gew.-%, bezogen auf das Gesamtgewicht des Katalysators, an Ruthenium und gegebenenfalls anderem Metall oder anderen Metallen der I-, VII- oder VIII-Nebengruppe auf den Träger aufgebracht vorliegen. Vorzugsweise beträgt diese Menge 0,2 bis 15 Gew.-%, besonders bevorzugt etwa 0,5 Gew.-%.

Die Metalloberfläche auf dem Katalysator beträgt insgesamt vorzugsweise 0,01 bis 10 m²/g, bevorzugt 0,05 bis 5 m²/g, insbesondere 0,05 bis 3 m²/g des Katalysators.

### TRÄGER

Die zur Herstellung der erfindungsgemäß verwendeten Katalysatoren verwendbaren Trägermaterialien sind vorzugsweise solche, die makroporös sind und einen mittleren Porendurchmesser von mindestens 0,1 µm, vorzugsweise mindestens 0,5 µm, und eine Oberfläche von höchstens 15 m²/g, aufweisen, vorzugsweise höchstens 10 m²/g, besonders bevorzugt höchstens 5 m²/g, insbesondere höchstens 3 m²/g. Bevorzugt liegt der mittlere Porendurchmesser des Trägers in einem Bereich von 0,1 bis 200 µm, insbesondere 0,5 bis 50 µm. Bevorzugt beträgt die Oberfläche des Trägers 0,2 bis 15 m²/g, besonders bevorzugt 0,5 bis 10 m²/g, insbesondere 0,5 bis 5 m²/g, speziell 0,5 bis 3 m²/g des Trägers.

Die Oberfläche des Trägers wird bestimmt nach dem BET-Verfahren durch N₂-Adsorption, insbesondere nach DIN 66131. Die Bestimmung des mittleren Porendurchmessers und der Porengrößenverteilung erfolgte durch Hg-Porosimetrie, insbesondere nach DIN 66133. Vorzugsweise kann die Porengrößenverteilung des Trägers annähernd bimodal sein, wobei die Porendurchmesserverteilung mit Maxima bei etwa 0,6 µm und etwa 20 µm bei der bimodalen Verteilung eine spezielle Ausführungsform der Erfindung darstellt.

Besonders bevorzugt ist ein Träger mit einer Oberfläche von etwa 1,75 m²/g, der diese bimodale Verteilung des Porendurchmessers aufweist. Das Porenvolumen dieses bevorzugten Trägers beträgt vorzugsweise etwa 0,53 ml/g.

Als makroporöses Trägermaterial verwendbar sind Aktivkohle, Siliciumcarbid, Aluminiumoxid, Siliciumdioxid, Titandioxid, Zirkoniumdioxid, Magnesiumoxid, Zinkoxid oder deren Gemische. Bevorzugt sind Aluminiumoxid und Zirkoniumdioxid.

Die erfindungsgemäß verwendeten Katalysatoren zeigen vorzugsweise eine hohe Reaktivität, Selektivität und Standzeit. Unter Einsatz der erfindungsgemäß verwendeten Katalysatoren werden in der Hydrierung die Hydrierungsprodukte vorzugsweise in hoher Ausbeute und Reinheit gewonnen.

### HYDRIERUNG

Die Hydrierung wird bei geeigneten Drücken und Temperaturen durchgeführt. Bevorzugt sind Drücke oberhalb von 50 bar, vorzugsweise von 150 bis 300 bar. Bevorzugte Temperaturen liegen in einem Bereich von 100 bis 270°C, vorzugsweise 150 bis 220°C.

### LÖSUNGS- ODER VERDÜNNUNGSMITTEL

Beim erfindungsgemäßen Verfahren kann die Hydrierung unter Abwesenheit eines Lösungs- oder Verdünnungsmittels durchgeführt werden, d.h. gemäß einer Ausführungsform ist es nicht erforderlich, die Hydrierung in Lösung durchzuführen. Vorzugsweise wird jedoch ein Lösungs- oder Verdünnungsmittel im erfindungsgemäßen Verfahren eingesetzt. Als Lösungs- oder Verdünnungsmittel kann jedes geeignete Lösungs- oder Verdünnungsmittel eingesetzt werden. Die Auswahl ist dabei nicht kritisch, beispielsweise können gemäß einer Ausführungsform die Lösungs- oder Verdünnungsmittel auch geringe Mengen an Wasser enthalten. Beispiele geeigneter Lösungs- oder Verdünnungsmittel schließen ein geradkettige oder cyclische Ether, wie beispielsweise Tetrahydrofuran oder Dioxan, sowie Ammoniak und Mono- oder Dialkylamine, in denen der Alkylrest vorzugsweise 1 bis 3 Kohlenstoffatome aufweist. Gemische dieser oder anderer Lösungs- oder Verdünnungsmittel können ebenfalls verwendet werden. Das Lösungs- oder Verdünnungsmittel kann in geeigneten Mengen verwendet werden, wobei solche Mengen bevorzugt sind, die zu einer 10- bis 70 gew.-%igen Lösung der zur Hydrierung vorgesehenen Verbindungen führen.

Als Lösungsmittel kann auch besonders bevorzugt das bei der Hydrierung nach dem erfindungsgemäßen Verfahren gebildete Produkt eingesetzt werden, gegebenenfalls neben anderen Lösungs- oder Verdünnungsmitteln. In diesem Fall kann ein Teil des im Hydrierungsverfahren gebildeten Produktes den zu hydrierenden Verbindungen beigemischt werden. Bezogen auf das Gewicht der zur Hydrierung vorgesehenen Verbindungen wird vorzugsweise die 1- bis 30fache, besonders bevorzugt die 5- bis 20fache Menge des Hydrierungsproduktes als Lösungs- oder Verdünnungsmittel zugemischt.

Vorzugsweise wird die Hydrierung in Gegenwart von Ammoniak oder Mono- oder Dialkylaminen, beispielsweise Methyl-, Ethyl-, Propylamin oder Dimethyl-, Diethyl-, Dipropylamin durchgeführt. Dabei werden geeignete Mengen an Ammoniak oder Mono- oder Dialkylamin eingesetzt, vorzugsweise 0,5 bis 50 Gewichtsteile, besonders bevorzugt 1 bis 20 Gewichtsteile, bezogen auf 100 Gewichtsteile der zur Hydrierung vorgesehenen Verbindung oder Verbindungen. Besonders bevorzugt werden wasserfreies Ammoniak oder wasserfreie Amine eingesetzt.

Das Hydrierungsverfahren kann kontinuierlich oder in Art eines Batch-Verfahrens durchgeführt werden.

Dabei kann bei kontinuierlicher Prozeßführung die Menge der zur Hydrierung vorgesehenen Verbindung bzw. Verbindungen vorzugsweise im Bereich von 0,05 bis 3 l pro Liter Katalysator pro Stunde betragen, vorzugsweise 0,1 bis 1 l pro Liter Katalysator pro Stunde.

Als Hydriergase können beliebige Gase verwendet werden, die freien Wasserstoff enthalten und keine schädlichen Mengen an Katalysatorgiften, wie beispielsweise CO, aufweisen. Beispielsweise können Reformerabgase verwendet werden. Vorzugsweise wird reiner Wasserstoff als Hydriergas verwendet.

Nachstehend wird die Erfindung anhand von Ausführungsbeispielen näher erläutert.

### BEISPIEL 1

### Herstellung des Katalysators

Ein makroporöser Aluminiumoxidträger in Form von 8 x 8 x 3 mm-Ringen, der eine Oberfläche von 1,75 m²/g, bestimmt nach dem BET-Verfahren, ein Porenvolumen von 0,531 ml/g sowie einen Porendurchmesser von 0,6 µm und 20 µm bei einer bimodalen Verteilung aufwies, wurde mit einer wäßrigen Ruthenium-(III)-nitrat-Lösung getränkt, die eine Konzentration von 0,7 bis 1 % Metall, bezogen auf das Gewicht der Lösung, aufwies. Das vom Träger aufgenommene Lösungsvolumen entsprach dabei in etwa dem Porenvolumen des verwendeten Trägers. Sodann wurde der mit der Ruthenium(III)-nitrat-Lösung getränkte Träger bei 120°C unter Bewegung getrocknet und bei 200°C im Wasserstoffstrom reduziert. Der so hergestellte Katalysator enthält 0,5 Gew.-% Ruthenium, bezogen auf das Gesamtgewicht des Katalysators, und weist eine Rutheniumoberfläche von 0,76 m²/g auf, bestimmt durch H₂-Puls-Chemiesorption (Puls Chemiesorp 2700, 35°C).

### HYDRIERUNG

1,2 l des nach vorstehender Vorschrift hergestellten Katalysators, der 0,5 Gew.-% Ruthenium auf einem makroporösen Al₂O₃-Träger enthielt, wurden in einen elektrisch beheizten Durchflußreaktor eingefüllt, der mit einem Abscheider ausgestattet war. Sodann wurde ohne vorhergehende Aktivierung des Katalysators eine Hydrierung von Anilin durchgeführt bei 230 bar und zunächst 160°C. Die Hydrierung wurde dabei kontinuierlich in Sumpffahrweise ausgeführt, wobei ein Teil des Hydrierungsaustrages rückgeführt wurde und dem Einsatzstoff vor dem Reaktor zugemischt wurde. Bezogen auf die Menge des eingesetzten Anilins wurde die 10fache Menge an Hydrierungsprodukt als Lösungsmittel zugesetzt. Am Kopf des Abscheiders wurden 500 bis 600l Wasserstoff pro Stunde entspannt. Die kontinuierlich dem Reaktor zugeführte Anilinmenge entsprach einer Katalysatorbelastung von 0,6 l/l Katalysator x h. In Abhängigkeit von der Reaktionstemperatur ergaben sich bei stationären Reaktionsbedingungen folgende Produktzusammensetzungen:

| **Temperatur °C** | **CHA**^{**1)**} **%** | **DCHA**^{**2)**} **%** | **Anilin %** | **Cyclohexan % + Cyclohexen** |
|---|---|---|---|---|
| 160 | 99,3 | 0,45 | 0,10 | 0,04 |
| 180 | 97,0 | 2,75 | 0,06 | 0,06 |
| 200 | 90,9 | 8,9 | - | 0,09 |

| | | | | |
|---|---|---|---|---|
| ¹⁾ CHA = Cyclohexylamin; | | | | |
| ²⁾ DCHA = Dicyclohexylamin | | | | |

### BEISPIEL 2

Es wurde eine Hydrierung wie in Beispiel 2 beschrieben durchgeführt, jedoch wurde zusätzlich kontinuierlich wasserfreies Ammoniak eindosiert. Bezogen auf 100 Gewichtsteile Anilin wurden 10 Gewichtsteile Ammoniak zugesetzt. In Abhängigkeit von der Reaktionstemperatur ergaben sich bei stationären Reaktionsbedingungen folgende Produktzusammensetzungen:

| **Temperatur °C** | **CHA**^{**1)**} **%** | **DCHA**^{**2)**} **%** | **Anilin %** | **Cyclohexan % + Cyclohexen** |
|---|---|---|---|---|
| 180 | 99,3 | 0,08 | 0,13 | 0,07 |
| 200 | 98,7 | 1,06 | - | 0,09 |

| | | | | |
|---|---|---|---|---|
| ¹⁾ CHA = Cyclohexylamin; | | | | |
| ²⁾ DCHA = Dicyclohexylamin | | | | |

### BEISPIEL 3

### Hydrierung von Toluylendiaminen

In einem 3,5 l fassenden Druckautoklaven wurden 2 kg einer 50 gew.-%igen Lösung von Toluylendiamin (Isomerengemisch von 2,4- und 2,6-Diaminotoluol) in THF vorgelegt sowie 500 ml des gemäß Beispiel 1 hergestellten Katalysators. Sodann wurde die Hydrierung diskontinuierlich bei einer Temperatur von 150°C und einem Druck von 200 bar für 5 Stunden durchgeführt. Der Umsatz zum entsprechenden cycloaliphatischen Diamin-Isomerengemisch war quantitativ, wobei der Restaromatengehalt kleiner als 0,01 % war.

### BEISPIEL 4

### Herstellung des Katalysators

Ein makroporöser Aluminiumoxidträger in Form von 8 x 8 x 3 mm-Ringen, der eine Oberfläche von 0,99 m²/g, bestimmt nach dem BET-Verfahren, ein Porenvolumen von 0,529 ml/g sowie einen Porendurchmesser von 1 µm und 30 µm bei einer bimodalen Verteilung aufwies, wurde mit drei Mal mit einer wäßrigen Nickel-(II)-nitrat-Lösung getränkt, die eine Konzentration von 13,5 % Metall, bezogen auf das Gewicht der Lösung, aufwies. Das vom Träger aufgenommene Lösungsvolumen entsprach dabei in etwa dem Porenvolumen des verwendeten Trägers. Nach jeder Tränkung wurde der getränkte Träger bei 120°C getrocknet und bei 520°C calciniert.

Der so hergestellte NiO/Aluminiumoxid-Katalysator wurde mit einer wäßrigen Ruthenium-(III)-nitrat-Lösung getränkt, die eine Konzentration von 0,5 bis 1 % Metall, bezogen auf Gewicht der Lösung aufwies. Das vom Träger aufgenommene Lösungsvolumen entsprach dabei in etwa dem Porenvolumen des verwendeten Trägers.

Anschließend wurde der mit der Ruthenium-(III)-nitrat-Lösung getränkte NiO/Aluminiumoxid-Katalysator bei 120°C unter Bewegung getrocknet und bei 200°C im Wasserstoffstrom reduziert. Der so hergestellte Katalysator enthält 0,5 Gew.-% Ruthenium und 11 % Nickel, bezogen auf das Gewicht des Katalysators.

### BEISPIEL 5

Analog Beispiel 1 wurde Anilin mit dem 0,5 % Ru/ 11 % Ni /Al₂O₃-Katalysator kontinuierlich in einem Durchflußreaktor umgesetzt. Bei 190°C und einer Katalysatorbelastung von 0,2 kg/l x h wurde bei vollständigem Umsatz eine Cyclohexylaminselektivität von 94% erzielt.

### BEISPIEL 6

Analog Beispiel 3 wurde Di-(4-aminophenyl)-methan in einem Autoklav quantitativ zu einem cis/trans-Gemisch von Di-(4-aminocyclohexyl)-methan umgesetzt.

## Patentansprüche

1. Verfahren zur Hydrierung von aromatischen Verbindungen, in denen mindestens eine Aminogruppe an einen aromatischen Kern gebunden ist, wobei man mindestens eine dieser Verbindungen in Gegenwart eines Katalysators mit freiem Wasserstoff in Berührung bringt, wobei der Katalysator Ruthenium und gegebenenfalls mindestens ein Metall der I-, VII- oder VIII-Nebengruppe in einer Menge von 0,01 bis 30 Gew.-%, bezogen auf das Gesamtgewicht des Katalysators, aufgebracht auf einen Träger, umfaßt, wobei der Träger einen mittleren Porendurchmesser von mindestens 0,1 µm und eine Oberfläche von höchstens 15 m²/g aufweist und ausgewählt ist aus Aktivkohle, Siliciumcarbid, Aluminiumoxid, Siliciumdioxid, Titandioxid, Zirkoniumdioxid, Magnesiumoxid, Zinkoxid oder deren Gemischen, vorzugsweise Aluminiumoxid oder Zirkoniumdioxid.

2. Verfahren nach Anspruch 1, das die folgenden Merkmale aufweist:
- die Menge des auf den Katalysator aufgebrachten Rutheniums und ggf. mindestens eines Metalls der I-, VII- oder VIII-Nebengruppe beträgt 0,2 bis 15 Gew.-%, bezogen auf das Gesamtgewicht des Katalysators,
- der mittlere Porendurchmesser des Trägers beträgt mindestens 0,5 µm
- die Porengrößenverteilung des Trägers ist annähernd bimodal,
- die Oberfläche des Trägers beträgt höchstens 10 m²/g,
- das mindestens eine Metall der I-, VII- oder VIII-Nebengruppe ist ausgewählt aus Kupfer, Rhenium, Kobalt und Nickel oder Gemischen davon,
- das auf den Träger aufgebrachte Metall weist eine Oberfläche von 0,01 bis 10 m² pro g, vorzugsweise 0,05 bis 5 m² pro g des Katalysators auf.

3. Verfahren nach Anspruch 1 oder 2, wobei der Katalysator aus einem makroporösen Aluminiumoxidträger mit einer Oberfläche von etwa 1,75 m²/g, einem Porenvolumen von etwa 0,53 ml/g, einer bimodalen Porengrößenverteilung mit Porendurchmessern von etwa 0,6 µm und etwa 20 µm besteht, auf den Ruthenium in einer Menge von etwa 0,5 Gew.-%, bezogen auf das Gesamtgewicht des Katalysators, aufgetragen ist, und wobei die Rutheniumoberfläche auf dem Träger etwa 0,76 m² pro g des Katalysators beträgt.

4. Verfahren nach einem der Ansprüche 1 bis 3, das die folgenden Merkmale aufweist:
- die aromatische Verbindung, in der mindestens eine Aminogruppe an einen aromatischen Kern gebunden ist, ist ein aromatisches Amin oder Diamin ist, vorzugsweise ausgewählt aus Anilin, Naphthylamin, Diaminotoluolen, Bis-p-Aminophenylmethan oder Gemischen davon,
- die Hydrierung in Gegenwart eines Lösungs- oder Verdünnungsmittels durchgeführt wird, vorzugsweise ausgewählt aus linearen oder cyclischen Ethern, vorzugsweise Tetrahydrofuran oder Dioxan, Ammoniak, oder Mono- oder Dialkylaminen, in denen der Alkylrest vorzugsweise 1 bis 3 Kohlenstoffatome aufweist, oder Gemischen davon,
- die Hydrierungsprodukt wird in Gegenwart des Hydrierungsproduktes durchgeführt, das in einer 1 bis 30fachen Menge, bezogen auf das Gewicht der zu hydrierenden Verbindung, vorliegt.

5. Katalysator, der Ruthenium und gegebenenfalls mindestens ein Metall der I-, VII- oder VIII-Nebengruppe in einer Menge von 0,01 bis 30 Gew.-%, bezogen auf das Gesamtgewicht des Katalysators, aufgebracht auf einen Träger, umfaßt, wobei der Träger einen mittleren Porendurchmesser von mindestens 0,1 µm und eine Oberfläche von höchstens 15 m²/g aufweist und ausgewählt ist aus Aktivkohle, Siliciumcarbid, Aluminiumoxid, Siliciumdioxid, Titandioxid, Zirkoniumdioxid, Magnesiumoxid, Zinkoxid oder deren Gemischen, vorzugsweise Aluminiumoxid oder Zirkoniumdioxid.

6. Katalysator nach Anspruch 5, dadurch gekennzeichnet, daß er wie in einem der Ansprüche 2 oder 3 definiert aufgebaut ist.

7. Verwendung eines Katalysators, nach Anspruch 5 oder 6 zur Hydrierung von Verbindungen, in denen mindestens eine Aminogruppe an einen aromatischen Kern gebunden ist.

8. Verwendung nach Anspruch 7, wobei die aromatische Verbindung, in der mindestens eine Aminogruppe an einen aromatischen Kern gebunden ist, ein aromatisches Amin oder Diamin ist.

9. Verwendung nach Anspruch 7 zur Hydrierung von Anilin, Naphthylamin, Diamintoluolen, Bis-p-Aminophenylmethan oder Gemischen davon.

## Claims

1. A process for hydrogenating aromatic compounds in which at least one amino group is bonded to an aromatic nucleus, wherein at least one of these compounds is brought into contact with free hydrogen in the presence of a catalyst, the catalyst comprises ruthenium and, if required, at least one metal of subgroup I, VII or VIII in an amount of from 0.01 to 30% by weight, based on the total weight of the catalyst, applied to a carrier, and the carrier has a mean pore diameter of at least 0.1 µm and a surface area of not more than 15 m²/g and is selected from active carbon, silicon carbide, alumina, silica, titanium dioxide, zirconium dioxide, magnesium oxide, zinc oxide and mixtures thereof, preferably alumina and zirconium dioxide.

2. A process as claimed in claim 1 which has the following features:
- ruthenium and, if required, at least one metal of subgroup I, VII or VIII are applied to the catalyst in an amount of from 0.2 to 15% by weight, based on the total weight of the catalyst,
- the mean pore diameter of the carrier is at least 0.5 µm,
- the pore size distribution of the carrier is approximately bimodal,
- the surface area of the carrier is not more than 10 m²/g,
- the one or more metals of subgroup I, VII or VIII are selected from copper, rhenium, cobalt and nickel and mixtures thereof,
- the metal applied to the carrier has a surface area of from 0.01 to 10, preferably from 0.05 to 5, m² per g of catalyst.

3. A process as claimed in claim 1 or 2, wherein the catalyst consists of a macroporous alumina carrier having a surface area of about 1.75 m²/g, a pore volume of about 0.53 ml/g, a bimodal pore size distribution with pore diameters of about 0.6 µm and about 20 µm, onto which carrier ruthenium is applied in an amount of about 0.5% by weight, based on the total weight of the catalyst, and the ruthenium surface area on the carrier is about 0.76 m² per g of catalyst.

4. A process as claimed in any of claims 1 to 3 which has the following features:
- the aromatic compound in which at least one amino group is bonded to an aromatic nucleus is an aromatic amine or diamine, preferably selected from aniline, naphthylamine, diaminotoluenes, bis-p-aminophenylmethane and mixtures thereof,
- the hydrogenation is carried out in the presence of a solvent or diluent, preferably selected from linear and cyclic ethers, preferably tetrahydrofuran and dioxane, ammonia and mono- and dialkylamines in which the alkyl radical is preferably of 1 to 3 carbon atoms, and mixtures thereof,
- the hydrogenation product is carried out in the presence of the hydrogenation product, which is present in an amount of from 1 to 30 times, based on the weight of the compound to be hydrogenated.

5. A catalyst which comprises ruthenium and, if required, at least one metal of subgroup I, VII or VIII in an amount of from 0.01 to 30% by weight, based on the total weight of the catalyst, applied to a carrier, wherein the carrier has a mean pore diameter of at least 0.1 µm and a surface area of not more than 15 m²/g and is selected from active carbon, silicon carbide, alumina, silica, titanium dioxide, zirconium dioxide, magnesium oxide, zinc oxide and mixtures thereof, preferably alumina and zirconium dioxide.

6. A catalyst as claimed in claim 5, which has a composition as defined in either of claims 2 or 3.

7. Use of a catalyst as claimed in claim 5 or 6 for hydrogenating compounds in which at least one amino group is bonded to an aromatic nucleus.

8. Use as claimed in claim 7, wherein the aromatic compound in which at least one amino group is bonded to an aromatic nucleus is an aromatic amine or diamine.

9. Use as claimed in claim 7 for hydrogenating aniline, naphthylamine, diaminotoluenes, bis-p-aminophenylmethane or mixtures thereof.

## Revendications

1. Procédé pour l'hydrogénation de composés aromatiques, dans lesquels au moins un groupe amino est fixé sur un noyau aromatique, dans lequel on met en contact avec de l'hydrogène libre au moins un de ces composés en présence d'un catalyseur, tandis que le catalyseur comprend du ruthénium et, éventuellement, au moins un métal du sous-groupe I, VII ou VIII en une quantité de 0,01 à 30% en poids, par rapport au poids total du catalyseur, déposé sur un support, tandis que le support présente un diamètre moyen des pore d'au moins 0,1 µm et une surface d'au plus 15 m²/g, et est choisi parmi le charbon actif, le carbure de silicium, l'oxyde d'aluminium, le dioxyde de silicium, le dioxyde de titane, le dioxyde de zirconium, l'oxyde de magnésium, l'oxyde de zinc ou leurs mélanges, de préférence l'oxyde d'aluminium ou le dioxyde de zirconium.

2. Procédé selon la revendication 1, qui présente les caractéristiques suivantes :
- la quantité du ruthénium présent sur le catalyseur et, éventuellement, d'au moins un métal du sous-groupe I, VII ou VIII, est de 0,2 à 15% en poids par rapport au poids total du catalyseur,
- le diamètre moyen des pores du support est d'au moins 0,5 µm,
- la distribution de la taille des pores du support est pratiquement bimodale,
- la surface du support est d'au plus 10 m²/g,
- le ou les métaux du sous-groupe I, VII ou VIII sont choisis parmi le cuivre, le rhénium, le cobalt et le nickel ou des mélanges de ceux-ci,
- le métal déposé sur le support présente une surface de 0,01 à 10 m² par gramme, de préférence de 0,05 à 5 m² par gramme du catalyseur.

3. Procédé selon la revendication 1 ou 2, dans lequel le catalyseur consiste en un support d'oxyde d'aluminium macroporeux ayant une surface d'environ 1,75 m²/g, un volume de pores d'environ 0,53 ml/g, une distribution bimodale de la taille des pores avec des diamètres de pores d'environ 0,6 µm et d'environ 20 µm, sur lequel est déposé du ruthénium en une quantité d'environ 0,5% en poids par rapport au poids total du catalyseur, et dans lequel la surface du ruthénium sur le support est d'environ 0,76 m² par gramme du catalyseur.

4. Procédé selon l'une des revendication 1 à 3, qui présente les caractéristiques suivantes :
- le composé aromatique, dans lequel au moins un groupe amino est fixé sur un cycle aromatique, est une amine ou une diamine aromatique, de préférence choisie parmi l'aniline, la naphtylamine, les diaminotoluènes, le bis-p-aminophénylméthane ou leurs mélanges,
- l'hydrogénation est effectuée en présence d'un solvant ou d'un diluant, de préférence choisi parmi les éthers linéaires ou cycliques, de préférence le tétrahydrofuranne ou le dioxanne, l'ammoniac, ou des mono- ou dialkylamines, dans lesquelles le reste alkyle présente 1 à 3 atomes de carbone, ou leurs mélanges,
- le produit d'hydrogénation est transformé en présence du produit d'hydrogénation qui est présent en une quantité de 1 à 30 fois, par rapport au poids du composé à hydrogéner.

5. Catalyseur qui comprend du ruthénium et éventuellement, un métal du sous-groupe I, VII ou VIII en une quantité de 0,01 à 30% en poids, par rapport au poids total du catalyseur, déposé sur un support, tandis que le support présente un diamètre moyen des pores d'au moins 0,1 µm et une surface d'au plus 15 m²/g et est choisi parmi le charbon actif, le carbure de silicium, l'oxyde d'aluminium, le dioxyde de silicium, le dioxyde de titane, le dioxyde de zirconium, l'oxyde de magnésium, l'oxyde de zinc ou leurs mélanges, de préférence l'oxyde d'aluminium ou le dioxyde de zirconium.

6. Catalyseur selon la revendication 5, caractérisé par le fait qu'il est élaboré comme défini dans l'une des revendications 2 ou 3.

7. Utilisation d'un catalyseur selon la revendication 5 ou 6 pour l'hydrogénation de composés dans lesquels au moins un groupe amino est fixé sur un cycle aromatique.

8. Utilisation selon la revendication 7, dans laquelle le composé aromatique, dans lequel au moins un groupe amino est fixé sur un cycle aromatique, est une amine ou une diamine aromatique.

9. Utilisation selon la revendication 7 pour l'hydrogénation d'aniline, de naphtylamine, de diaminotoluènes, de bis-p-aminophénylméthane ou de leurs mélanges.
